# EUROPEAN PATENT APPLICATION

(11) **EP 3 965 114 A1**
(43) Date of publication of application: **09.03.2022**
(21) Application number: 21194429.3
(22) Date of filing: 01.09.2021
(51) Int. Cl.: G16H 20/30

(54) **PRIVACY PRESERVING PERSONALIZED WORKOUT RECOMMENDATIONS**

(30) Priority: 03.09.2020 US 202063074374 P
(71) Applicant: Apple Inc., Cupertino, CA 95014 (US)
(72) Inventor: Bedekar, Niharika M., Cupertino, 94014 (US); Angelone, Joel J., Cupertino, 94014 (US); Arney, Julie A., Cupertino, 94014 (US); Foo, Jeremy Jie You, Cupertino, 94014 (US); Clark, Davis S., Cupertino, 94014 (US); Panesar, Parry, Cupertino, 94014 (US); Blahnik, Jay K., Cupertino, 94014 (US); Lareau, Brett L., Cupertino, 94014 (US)
(74) Representative: Barton, Russell Glen

(57) **Abstract**

In some implementations, a computing system can be configured for presenting privacy preserving personalized workout recommendations. In some implementations, a workout application of system 100 can generate workout recommendations based on workouts previously performed by a user within the workout application and/or external to the workout application. For example, a user can participate in an internal workout presented by the workout application or an external workout performed without the aid of the workout application and detected by one or more sensors of a user device carried by the user during the external workout. The various attributes of the workouts can be stored as historical workout data and used by the workout application to recommend to the user workouts available through the workout application, or corresponding workout service. The workout recommendations can be generated on the user device to preserve the privacy of the user's personal health information.

## Description

### TECHNICAL FIELD

The disclosure generally relates to generating and presenting content recommendations in a privacy preserving manner.

### BACKGROUND

Many modern computing devices track health and fitness data using various sensors and/or applications on a user device. Some computing devices even provide access to workouts or exercise content. However, keeping users engaged in a workout routine or workout service may be difficult when the user is not exposed to new workouts or workout content.

### SUMMARY

In some implementations, a computing system can be configured for presenting privacy preserving personalized workout recommendations. In some implementations, a workout application of system 100 can generate workout recommendations based on workouts previously performed by a user within the workout application and/or external to the workout application. For example, a user can participate in an internal workout presented by the workout application or an external workout performed without the aid of (or workout content provided by) the workout application and detected by one or more sensors of a user device carried by the user during the external workout. The various attributes of the workouts can be stored as historical workout data and used by the workout application to recommend to the user workouts available through the workout application, or corresponding workout service. The workout recommendations can be generated on the user device to preserve the privacy of the user's personal health information.

Particular implementations provide at least the following advantages. Workout content recommendations can be intelligently selected and/or personalized for a particular user thereby reducing the burden on the user to search for and select workout content that the user may enjoy or that might align with the user's exercise goals. Moreover, by selecting workout content based on user specific information, workout history, etc., the system can reduce the amount of network bandwidth, processing cycles, and power (e.g., battery) needed to present workout content that the user may never view.

Details of one or more implementations are set forth in the accompanying drawings and the description below. Other features, aspects, and potential advantages will be apparent from the description and drawings, and from the claims.

### DESCRIPTION OF DRAWINGS

FIG. 1 is a block diagram of an example system for presenting privacy preserving personalized workout recommendations.
FIG. 2 is a block diagram of an example system for presenting suggested workouts by a user playback device.
FIG. 3 is a block diagram of a system for presenting suggested workouts at a communal playback device that were generated by a wearable device.
FIG. 4 is a block diagram of an example system for presenting suggested workouts by a wearable device.
FIG. 5 is a block diagram of an example system for generating workout recommendations.
FIG. 6 is an example table associating workout focuses areas with workout types and complementary workout areas.
FIG. 7 illustrates an example graphical user interface for presenting recommended workouts.
FIG. 8 is flow diagram of an example process for privacy preserving personalized workout recommendations.
FIG. 9 is a block diagram of an example computing device that can implement the features and processes of FIGS. 1-8.

Like reference symbols in the various drawings indicate like elements.

### DETAILED DESCRIPTION

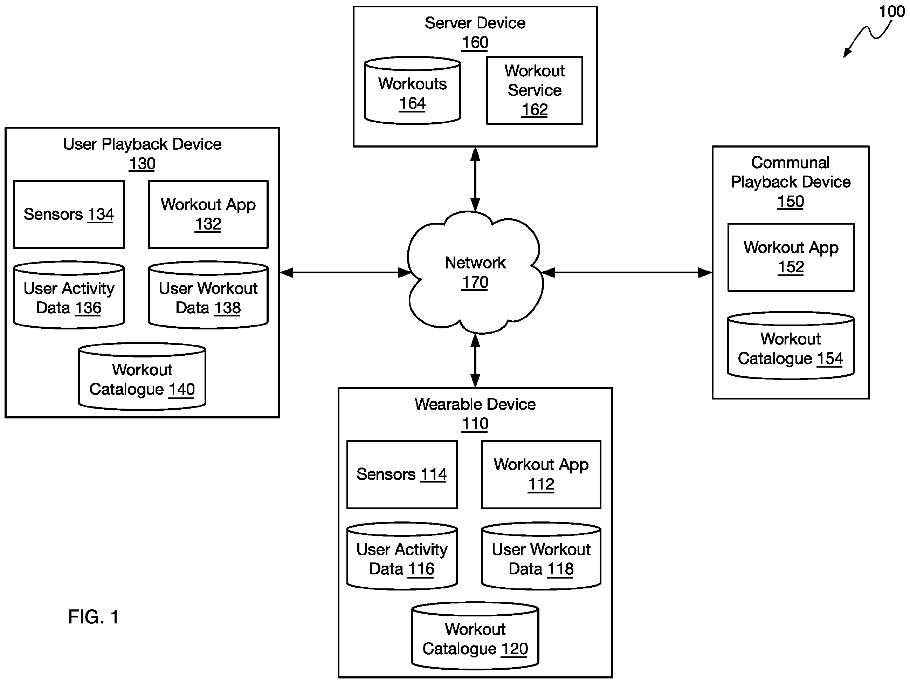

FIG. 1 is a block diagram of an example system 100 for presenting privacy preserving personalized workout recommendations. System 100 can include various user computing devices (e.g., wearable device 110, user playback device 130, communal playback device 150) that can be configured to generate and/or present workout recommendations from a catalogue of workouts served by a server computing device (e.g., server device 160). Each of the computing devices in system 100 can communicate with each other, as described below, through various networks represented in FIG. 1 by network 170 (e.g., the Internet, wide area network, local area network, wireless network, Bluetooth network, cellular data network, direct peer-to-peer network, etc.). For example, the user computing devices may communicate with each other directly using a Bluetooth connection or local area network. The user computing devices may communicate with server device 160 through a local area network, a wide area network, the Internet, and the like.

In some implementations, system 100 can include wearable device 110. For example, wearable device 110 can be a computing device, such as a smart watch, smart glasses, smart clothing, etc. Wearable device 110 can be include workout application 112. For example, workout application 112 can be a client application of a network workout service (e.g., workout service 162 of server device 160) configured to serve workout data (e.g., workouts, workout content, workout metadata, a catalogue of workouts, etc.) workout application 112 for presentation to the user. For example, workout application 112 can receive a catalogue of workouts from the server device, select workouts from the catalogue for recommendation to the user, and send the recommendations to another computing device (e.g., communal playback device 150) for presentation to the user.

In some implementations, workout application 112 can present the recommendations on a display of wearable device 110. For example, workout application 112 can present the selected workout recommendations and present the workout recommendations on the display of wearable device 110. Wearable device 110 can receive user input selecting a recommended workout and present the content of the workout on a display of wearable device 110. However, due to the fact that wearable device 110 typically (although not always) has a small form factor, wearable device 110 may cause workout content to be presented by another playback device, such as communal playback device 150, as described further below.

In some implementations, workout application 112 can store data identifying workouts performed by the user in user workout database 118. For example, when a user selects a recommended workout, or other workout, workout application 112 can store workout data (e.g., workout attributes) describing the user's performance of the selected workout in user workout database 118. For example, the record for each workout performed by the user can include a workout identifier, a workout type, a workout category, a workout duration, identifiers for trainers (e.g., people) involved in the workout, identifiers for genres music included in the workout content, and/or a content type identifier. The workout type (workout modality) can correspond to specific exercises, such as cycling, running, walking, resistance training, HIlT (high intensity interval training), etc. The workout category can correspond to the effect of the workout, such as cardiovascular (cardio) training, strength training, flexibility training, core (e.g., abdominal, lower back, stabilizer muscles, etc.) training, etc. The content type can identify whether the workout content is audio/video content or audio-only content. The workout duration can indicate an amount of time the user spent performing the workout.

In some implementations, wearable device 110 can be configured to detect and store user activity data related to workouts performed external to workout application 112. For example, due to the fact that wearable device 110 is designed to be worn by the user, wearable device 110 may be ideally situated to monitor user physical activity of a user who is wearing wearable device 110. Thus, wearable device 110 may include sensors 114 (e.g., motion sensors, heartrate sensors, etc.) that may detect and generate data describing various user activities while wearable device 110 is worn by the user. This sensor data and/or other context data (e.g., location of wearable device 110, weather near wearable device 110, etc.) can be stored as user activity data in user activity database 116. In some implementations, the sensor data and/or context data in user activity database 116 can be analyzed by wearable device 110 to determine specific workout activities (e.g., running, walking, biking, etc.) performed by the user and determine patterns of workout activities (e.g., the user runs every day at 9am when not raining). These workout activities and/or workout patterns can be stored as user activity data in user activity database 116 along with the corresponding sensor and context data.

In some implementations, wearable device 110 can include workout catalogue database 120. For example, workout application 112 can communicate with workout service 162 on server device 160 to obtain a catalogue of workouts provided by (e.g., available through) workout service 162. Workout application 112 can obtain the catalogue of workouts from workout service 162 when workout application 112 is invoked and a network connection to workout service 162 is available, for example. Alternatively, workout application 112 can periodically (e.g., every 24 hours, every 72 hours, once a week, etc.) obtain the workout catalogue and store the workout catalogue in workout catalogue database 120.

In some implementations, workout application 112 can store data describing workouts in the workout catalogue in workout catalogue database 120. For example, the record for each workout in workout catalogue database 120 can include a workout identifier, a workout type, a workout category, a workout duration, identifiers for trainers (e.g., people) involved in the workout, identifiers for genres music included in the workout content, and/or a content type identifier. The workout type can, for example, correspond to specific exercises, such as cycling, running, walking, resistance training, HIlT (high intensity interval training), etc. The workout category can, for example, correspond to the training effect of the workout, such as cardiovascular (cardio) training, strength training, flexibility training, core (e.g., abdominal, lower back, stabilizer muscles, etc.) training, etc. The content type can, for example, identify whether the workout content is audio/video content or audio-only content. The workout duration in the catalogue of workouts can indicate an amount of time the workout is intended to take, or the amount of time needed to present the workout content from beginning to end.

In some implementations, system 100 can include user playback device 130. For example, user playback device 130 can be a computing device, such as a smart phone, tablet computer, laptop computer, or other computing device having a display large enough to present content comfortably viewable by a user. User playback device 130 (and wearable device 110) is distinguishable from communal playback device 150 in that user playback device 130 is configured as a single user device or intended to require individuals to login to separate user accounts to use user playback device 130, while communal playback device 150 may be configured with a single user account that many different users use to access the features of communal playback device 150. Thus, user playback device 140 (and wearable device 110) may offer security for private personal information (e.g., health data, location data, workout data, etc.), while data stored on communal playback device 150 may be accessible to anyone using communal playback device 150. Therefore, user playback device 130 and wearable device 110 may store and exchange a user's private personal information and use the private personal information to generate workout recommendations, but communal playback device 150 will not have access to the user's personal private information and will not be able to generate workout recommendations, as described herein.

In some implementations, user playback device 130 can be configured similarly to wearable device 110. For example, other than the differences in form factor (e.g., size, wearability, display size, etc.) and types of sensors, user playback device 130 can be configured similarly to wearable device 110. For example, since user playback device 130 will often have a larger display than wearable device 110, user playback device 130 may be configured to present workout suggestions and/or workout content using the display of user playback device 130.

In some implementations, user playback device 130 can be include workout application 132. For example, workout application 132 can be a client application of a network workout service (e.g., workout service 162 of server device 160) configured to serve workout data (e.g., workouts, workout content, workout metadata, a catalogue of workouts, etc.) workout application 132 for presentation to the user. For example, workout application 132 can receive a catalogue of workouts from the server device, select workouts from the catalogue for recommendation to the user, and present the recommendations to the user on a display of user playback device 130. Workout application 132 can receive user input selecting a recommended workout and present the content (e.g., audio/video, audio only, etc.) of the workout on a display of user playback device 130.

In some implementations, workout application 132 can store data identifying workouts performed by the user in user workout database 138. For example, when a user selects a recommended workout, or other workout, workout application 132 can store workout data (e.g., workout attributes) describing the user's performance of the selected workout in user workout database 138. For example, the record for each workout performed by the user can include a workout identifier, a workout type, a workout category, a workout duration, identifiers for trainers (e.g., people) involved in the workout, identifiers for genres music included in the workout content, and/or a content type identifier. The workout type can correspond to specific exercises, such as cycling, running, walking, resistance training, HIIT (high intensity interval training), etc. The workout category can correspond to the effect of the workout, such as cardiovascular (cardio) training, strength training, flexibility training, core (e.g., abdominal, lower back, stabilizer muscles, etc.) training, etc. The content type can identify whether the workout content is audio/video content or audio-only content. The workout duration can indicate an amount of time the user spent performing the workout.

In some implementations, user playback device 130 can be configured to detect and store user activity data related to workouts performed external to workout application 132. For example, user playback device 130 may include sensors 134 (e.g., motion sensors) that may detect and generate data describing various user activities while user playback device 130 is carried by the user. This sensor data and/or other context data (e.g., location of user playback device 130, weather near user playback device 130, etc.) can be stored as user activity data in user activity database 136. In some implementations, the sensor data and/or context data in user activity database 136 can be analyzed by user playback device 130 to determine specific workout activities (e.g., running, walking, biking, etc.) performed by the user and determine patterns of workout activities (e.g., the user walks every day at 8am when temperature is above 90 degrees). These workout activities and/or workout patterns can be stored as user activity data in user activity database 136 along with the corresponding sensor and context data.

In some implementations, user playback device 130 can include workout catalogue database 140. For example, workout application 132 can communicate with workout service 162 on server device 160 to obtain a catalogue of workouts provided by (e.g., available through) workout service 162. Workout application 132 can obtain the catalogue of workouts from workout service 162 when workout application 132 is invoked and a network connection to workout service 162 is available, for example. Alternatively, workout application 132 can periodically (e.g., every 24 hours, every 72 hours, once a week, etc.) obtain the workout catalogue and store the workout catalogue in workout catalogue database 140.

In some implementations, workout application 132 can store data (e.g., workout attributes) describing workouts in the workout catalogue in workout catalogue database 140. For example, the record for each workout in workout catalogue database 140 can include a workout identifier, a workout type, a workout category, a workout duration, identifiers for trainers (e.g., people) involved in the workout, identifiers for genres music included in the workout content, and/or a content type identifier. The workout type can, for example, correspond to specific exercises, such as cycling, running, walking, resistance training, HIIT (high intensity interval training), etc. The workout category can, for example, correspond to the training effect of the workout, such as cardiovascular (cardio) training, strength training, flexibility training, core (e.g., abdominal, lower back, stabilizer muscles, etc.) training, etc. The content type can, for example, identify whether the workout content is audio/video content or audio-only content. The workout duration in the catalogue of workouts can indicate an amount of time the workout is intended to take, or the amount of time needed to present the workout content from beginning to end.

In some implementations, user activity data 116/136, user workout data 118/138, and/or workout catalogue data 120/140 can be synchronized between wearable device 110 and user playback device 130. As the data is updated or modified, the updates and/or modifications can be synchronized between wearable device 110 and user playback device 130. The data can be synchronized over a direct peer-to-peer connection (e.g., Bluetooth, peer-to-peer Wi-Fi, etc.). The data can be synchronized over a network connection (e.g., local area network, wide area network, Internet, etc.). The data can be synchronized using a secure cloud storage account associated with the user of wearable device 110 and user playback device 130, for example.

In some implementations, system 100 can include communal playback device 150. For example, communal playback device 150 can be a computing device (e.g., a set-top-box, smart speaker, smart television, etc.) that is shared amongst several different users. In some instances, communal playback device 150 may be configured for a specific user account but left unsecure and accessible to any user who has access to an input device configured to provide input to communal playback device 150. Thus, system 100 may be configured to avoid sending private personal information (e.g., health data, user activity data, user location data, etc.) to communal playback device 150.

In some implementations, communal playback device 150 can include workout application 152. For example, workout application 152 can be configured to present workout recommendations at communal playback device 150 and present workout content. However, unlike workout applications 112/132, workout application 152 will not generate personalized workout recommendations since workout application 152 does not have access to the user's private personal information (e.g., health data, workout history, etc.).

In order to present personalized workout recommendations, workout application 152 can receive personalized workout recommendations from wearable device 110 and/or user playback device 130. For example, the recommendation can include, for each workout, a workout identifier and a description of the reason why the workout was recommended for the user. After receiving the recommendations, workout application 152 can present the workout recommendations. For example, like wearable device 110 and/or user playback device 130, server device 150 can receive a workout catalogue from workout service 162 on server device 160 that describes the various workouts available through workout service 162. The workout catalogue can include workout metadata (e.g., workout descriptions, images, durations, etc.) describing various attributes of the available workouts, as described above. Workout application 152 can store the workout catalogue in workout catalogue database 154. When workout application 152 receives workout recommendations from wearable device 110 and/or user playback device 130, workout application 152 can use the workout identifiers in the workout recommendations to obtain metadata for workouts corresponding to the workout identifiers. Workout application 152 can present the workout metadata and an indication of why the workout was recommended on a display of communal playback device 150.

In some implementations, workout application 152 can receive user input selecting a workout. For example, workout application 152 can receive user input from an input device (e.g., remote control device, wearable device 110, user playback device 130) selecting a recommended workout presented by communal playback device 150. Workout application 152 can then request the content for the selected workout from workout service 162 by sending the workout identifier of the selected workout to workout service 162. When the workout content is received from workout service 162, workout application 152 can present the workout content using the display and/or speakers of communal playback device 150.

In some implementations, system 100 can include server device 160. For example, server device 160 can be a network computing device configured to serve workout data (e.g., workout content, workout metadata, workout catalogues, etc.) to client devices (e.g., wearable device 110, user playback device 130, communal playback device 150, etc.). Server device 160 can include workout service 162. For example, workout service 162 can be a software server running on server device 160 and configured to serve workout data to client devices. Server device 160 can include workouts database 164. For example, workouts database 164 can store workout data for the workouts available through workout service 162.

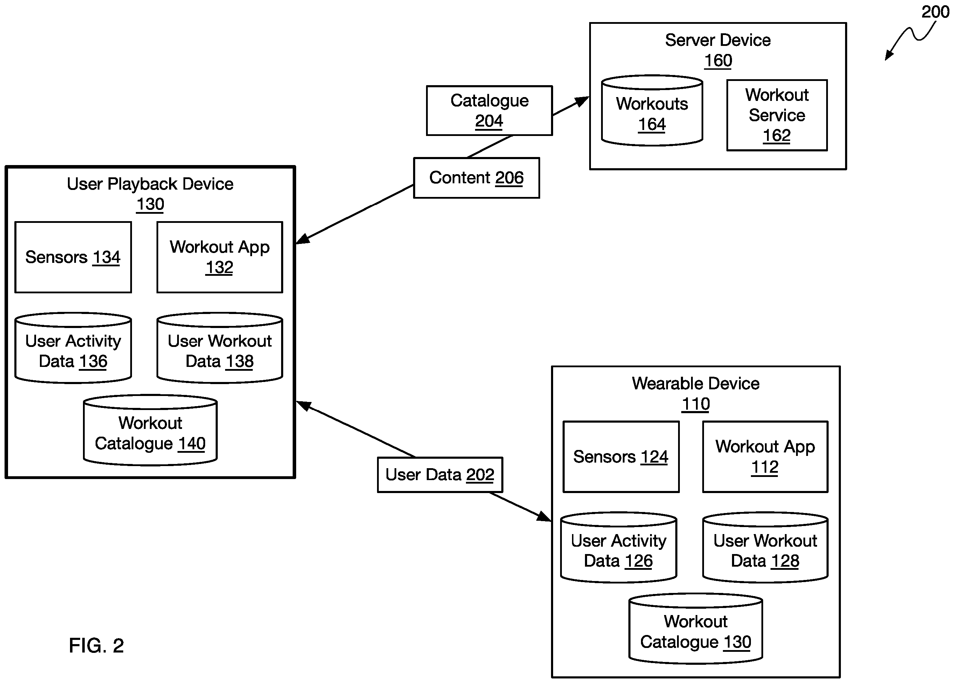

FIG. 2 is a block diagram of an example system 200 for presenting suggested workouts by a user playback device. For example, system 200 can correspond to system 100. However, system 200 highlights an example use case where user playback device 130 generates and presents suggested workouts for a user of user playback device 130 and/or wearable device 110. Since user playback device 130 is a personal computing device of an authenticated user, user playback device 130 may store private personal information that workout application 132 may use to generate workout suggestions. Since the workout suggestions are generated on user playback device 130, the user's private personal information may be protected from malicious external actors who may gain access to the user's information if the user's private personal information were sent to server device 160.

As described above, user playback device 130 and wearable device 110 can share user data 202. For example, user data 202 (e.g., user activity data 126/136, user workout data 128/138) can be synchronized between user playback device 130 and wearable device 110. Workout application 132 can use the user data to determine workouts that the user has previously performed within (e.g., internal workouts) workout application 132/112 and outside (e.g., external workouts) of workout application 132/112. Workout application 132 can obtain workout catalogue 204 from workout service 162 on server device 160 and store the workout catalogue in workout catalogue database 140. Workout application 132 can generate recommended workouts based on the determined internal workouts and external workouts and the workouts in workout catalogue database 140. For example, in response to receiving user input invoking workout application 132, workout application 132 may generate workout recommendations based on the user's past workouts. Workout application 132 can present the recommended workouts and/or a description of why the workouts are recommended, to the user of playback device 130.

After presenting the recommended workouts, workout application 132 can receive user input selecting a workout (e.g., a recommended workout) and send an identifier for the selected workout to workout service 162. Workout service 162 can send workout content 206 (e.g., audio/video content, audio-only content, etc.) corresponding to the identified workout to workout application 132. For example, workout content 206 can be streamed to workout application 132 or downloaded (e.g., as a whole, or in part) to workout application 132. Workout application 132 can present the workout content 206 in response to receiving user input to initiate the presentation of workout content 206.

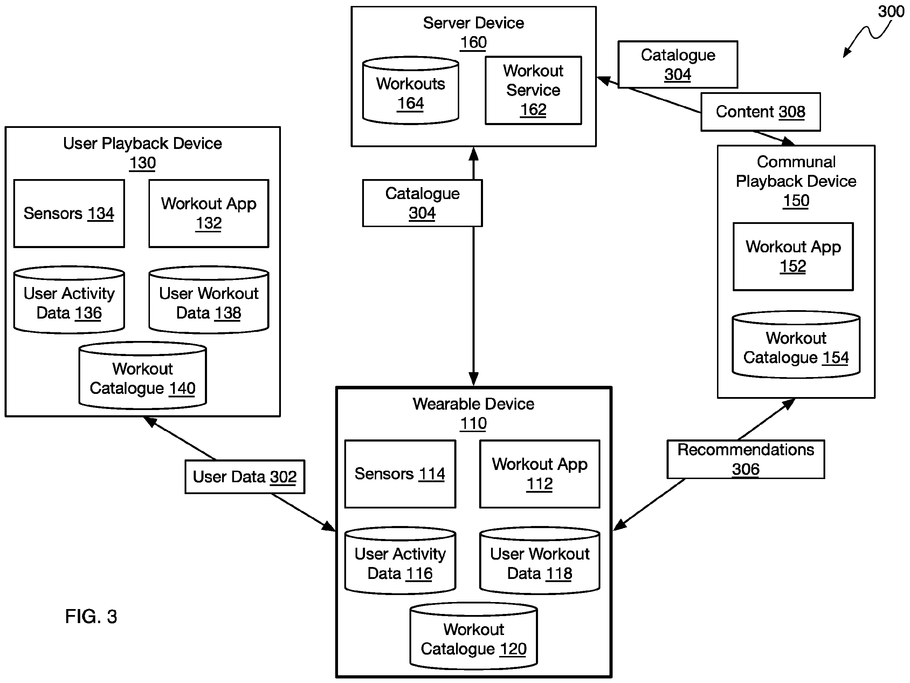

FIG. 3 is a block diagram of a system 300 for presenting suggested workouts at a communal playback device that were generated by a wearable device. For example, system 200 can correspond to system 100. However, system 300 highlights an example use case where wearable device 110 generates suggested workouts for a user of wearable device 110 and sends the recommended workouts to communal playback device 150 for presentation to the user. A similar approach can be used to generate workout recommendations at user playback device 130 (e.g., if wearable device 110 is unavailable) and send the workout recommendations to communal playback device 150 when the user wishes to perform a workout using communal playback device 150. Since user wearable device 110 is a personal computing device of an authenticated user, wearable device 110 may store private personal information that workout application 112 may use to generate workout recommendations. Since the workout recommendations are generated on wearable device 110, the user's private personal information may be protected from malicious actors who may gain access to the user's information if the user's private personal information were sent to server device 160 or communal playback device 150.

As described above, user playback device 130 and wearable device 110 can share user data 202. For example, user data 202 (e.g., user activity data 126/136, user workout data 128/138) can be synchronized between user playback device 130 and wearable device 110. Workout application 112 on wearable device 110 can use the user data to determine workouts that the user has previously performed within (e.g., internal workouts) workout application 132/112 and outside (e.g., external workouts) of workout application 132/112.

Workout application 112 on wearable device 110 can obtain workout catalogue 304 from workout service 162 on server device 160 and store the workout catalogue in workout catalogue database 120. Similarly, workout application 152 on communal device 150 can obtain workout catalogue 304 from workout service 162 on server device 160 and store the workout catalogue in workout catalogue database 154.

In some implementations, workout application 112 on wearable device 110 can generate recommended workouts based on the determined internal workouts and external workouts that the user has previously performed and the workout data in workout catalogue database 120. For example, workout application 112 can generate recommended workouts for a user of wearable device 110 in response to a request for workout recommendations received from workout application 152 on communal playback device 150. For example, the user of wearable device 110 may invoke workout application 152 on communal playback device 150 in anticipation of performing a workout presented by communal playback device 150. In response to receiving the invocation of workout application 152 (e.g., during initialization of workout application 152), workout application 152 can send a message to wearable device 110 requesting workout recommendations.

When wearable device 110 receives the message requesting workout recommendations, wearable device can generate and send workout recommendations 306 to workout application 152 on communal playback device 150. For example, the workout data for each workout included in workout recommendations 306 can include a workout identifier and a description of why the workout is being recommended to the user. After generating the workout recommendations 306, workout application 112 can send workout recommendations 306 to workout application 152 on communal playback device 150. For example, since the workout recommendations 306 sent to communal playback device 150 exclude private personal data, system 300 can avoid exposing the user's private personal data to other unauthorized users or malicious actors.

In some implementations, workout application 152 on communal device 150 can present workout suggestions received from wearable device 110 (or user playback device 130). For example, workout application 152 can present the recommended workouts and/or a description of why the workouts are recommended, to the user of playback device wearable device 110 and/or communal playback device 150. Workout application 152 can receive user input selecting a workout (e.g., a recommended workout) and send an identifier for the selected workout to workout service 162 in a request for content corresponding to the selected workout. Workout service 162 can send workout content 308 (e.g., audio/video content, audio-only content, etc.) corresponding to the identified workout to workout application 152. For example, workout content 308 can be streamed to workout application 152 or downloaded (e.g., as a whole, or in part) to workout application 152. Workout application 152 can present the workout content 308 in response to receiving user input to initiate the presentation of workout content 308.

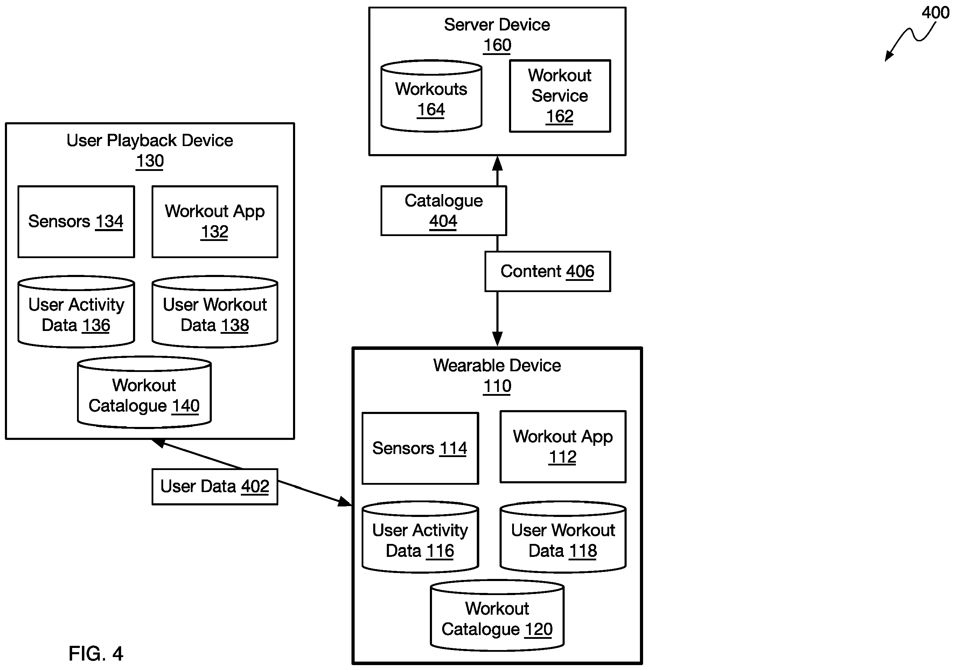

FIG. 4 is a block diagram of an example system 400 for presenting suggested workouts by a wearable device. For example, system 400 can correspond to system 100. However, system 400 highlights an example use case where wearable device 110 generates and presents suggested workouts for a user of wearable device 110 and/or user playback device 130. Since wearable device 110 is a personal computing device of an authenticated user, wearable device 110 may store private personal information that workout application 112 may use to generate workout suggestions. Since the workout suggestions are generated on wearable device 110 (e.g., on device), the user's private personal information may be protected from malicious external actors who may gain access to the user's information if the user's private personal information were sent to server device 160.

As described above, user playback device 130 and wearable device 110 can share user data 402. For example, user data 402 (e.g., user activity data 126/136, user workout data 128/138) can be synchronized between user playback device 130 and wearable device 110. Workout application 112 can use the user data to determine workouts that the user has previously performed within (e.g., internal workouts) workout application 132/112 and outside (e.g., external workouts) of workout application 132/112. Workout application 112 can obtain workout catalogue 404 from workout service 162 on server device 160 and store the workout catalogue 404 in workout catalogue database 120. Workout application 112 can generate recommended workouts based on the determined internal workouts and external workouts and the workouts in workout catalogue database 120. For example, in response to receiving user input invoking workout application 112, workout application 112 may generate workout recommendations based on the user's past workouts. Workout application 112 can present the recommended workouts and/or a description of why the workouts are recommended, to the user of wearable device 110. For example, the recommended workouts may be presented on a display of wearable device 110. However, since wearable device 110 likely has a small display, if any, the recommended workouts may be presented using audio data (e.g., text to voice) and the user may perform workout selections by providing voice input to wearable device 110.

After presenting the recommended workouts, workout application 112 can receive user input selecting a workout (e.g., a recommended workout) and send an identifier for the selected workout to workout service 162. Workout service 162 can send workout content 406 (e.g., audio/video content, audio-only content, etc.) corresponding to the identified workout to workout application 112. For example, workout content 406 can be streamed to workout application 112 or downloaded (e.g., as a whole, or in part) to workout application 112. Workout application 112 can present the workout content 406 in response to receiving user input to initiate the presentation of workout content 406. For example, since wearable device 110 likely has a small form factor and/or small display, if any, the workout content 406 received from workout service 162 may be audio-only content configured specifically for presentation by wearable devices like wearable device 110. Thus, when requesting workout content 406, wearable device 110 may include both the workout identifier of the selected workout and a device type identifier for wearable device 110 so that workout service 162 can select the appropriate workout content for wearable device 110.

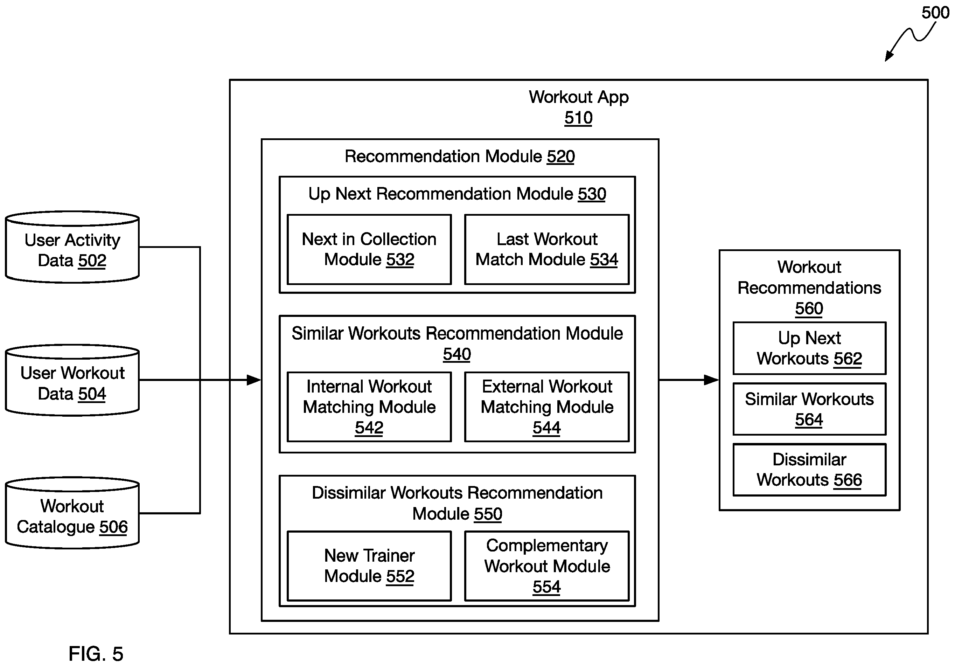

FIG. 5 is a block diagram of an example system 500 for generating workout recommendations. For example, system 500 can correspond to system 100, described above. Thus, user activity data 502 may correspond to user activity data 116/136. User workout data 504 may correspond to user workout data 118/138. Workout catalogue 506 may correspond to workout catalogue 120/140/154. Workout application 510 may correspond to workout application 112/132.

In some implementations, workout application 510 can include recommendation module 520. For example, recommendation module 520 can be a software module configured to generate various types of workout recommendations from workout catalogue database 506 based on internal and/or external workouts that the user has previously performed (as indicated by the historical workout data stored in user activity database 502 and/or user workout database 504) and various workout selection criteria, as described further below. A general selection criterion being that recommendation module 520 may not select for recommendation a workout from the workout catalogue that the user has previously performed.

In some implementations, recommendation module 520 can include "up next" recommendation module 530. For example, up next recommendation module 530 can recommend workouts that are next in a collection or series of workouts where the user has previously performed one or more workouts, excluding the last workout, in the workout collection or series. As another example, up next recommendation module 530 can recommend workouts that have attributes that are identical to the last workout performed by the user.

In some implementations, up next recommendation module 522 can include a "next in collection" module 532. For example, next in collection module 532 can select workouts from workout catalogue database 506 that are next in a collection (e.g., series, program, etc.) of workouts where the user has previously performed one or more workouts in the collection in the workout collection. For example, some workouts in workout catalogue can be organized into a workout collection, series, or program. For example, a series of workouts can be organized into a workout program that, as the user progresses through the workout program, improves the user's flexibility, strength, or other fitness goal.

Next in collection module 532 can select a workout for recommendation to the user based on a set of criteria. For example, module 532 can select a workout from a particular workout program in workout catalogue database 506 when the user has completed another workout from the particular workout program within the last 14 days. Module 532 can select a workout from a particular workout program in workout catalogue database 506 when the user has completed more than a threshold number of minutes (e.g., 3 minutes, 5 minutes, 10 minutes, etc.) of the previously performed (e.g. last performed) workout in the particular workout program. Module 532 can select a workout from a particular workout program in workout catalogue database 506 when the user has not completed the last workout in a series of workouts in the particular workout program. For example, if the particular workout program has a series of 6 workouts and the user has completed the sixth (e.g., last workout), the workout program can be considered completed and another workout from the particular workout program will not be recommended. In some implementations, next in collection module 532 may select up to a maximum number (e.g., 2, 3, 4, etc.) of workouts to recommend to the user.

In some implementations, up next recommendation module 522 can include a "last workout match" module 534. For example, module 534 can select workouts from workout catalogue 506 that match certain criteria of the last workout performed by the user. As described above, user workout database 504 can store data (e.g., workout attributes) describing workouts provided by workout application 510 the user has previously performed. Module 534 can determine the last (e.g., most recent) workout performed by the user in user workout database 504 and determine the workout attribute values for the time (e.g., timestamp indicating when the workout was performed), trainer, workout type, and duration attributes of the last performed workout.

In some implementations, module 534 can select workouts from workout catalogue 506 based on the last performed workout when the last performed workout was performed within a previous period of time (e.g., last 30 days, last 45 days, last 2 weeks, etc.). Module 534 can select workouts from workout catalogue 506 based on the last performed workout when the user completed a threshold amount (e.g., 3 minutes, 5 minutes, 15 minutes, etc.) of the last performed workout. Module 534 can select workouts from workout catalogue 506 based on the last performed workout when the last performed workout is not part of a collection of workouts (e.g., workout series, workout program, etc.).

In some implementations, module 534 can select workouts from workout catalogue 506 based on the last performed workout when a workout in workout catalogue database 506 matches the trainer, workout type, and duration attribute values of the last performed workout. For example, the trainer attribute value of a selected workout can match the trainer attribute value of the last performed workout when the trainer associated with the last performed workout is one of the trainers associated with the selected workout. The workout type attribute value of a selected workout can match the workout type attribute value of the last performed workout when the workout type associated with the last performed workout is the same as the workout type associated with the selected workout. The duration attribute value of a selected workout can match the duration attribute value of the last performed workout when the duration associated with the last performed workout is within a threshold number of minutes (e.g., 5 minutes, 7 minutes, 10 minutes, etc.) of the duration associated with the selected workout.

In some implementations, up next recommendation module 530 can receive the workout identifiers and selection reasons for workouts selected by module 532 and/or module 534 and generate up next workout recommendations 562. For example, the selection reasons generated by next in collection module 532 can indicate that the selected workout is the next in a series (e.g., collection, program, etc.) of workouts that the user has started. The selection reasons generated by last workout match module 534 can indicate that the selected workout has some of the same attributes as the last workout performed by the user. Up next workout recommendations 562 can be provided to recommendation module 520 so that recommendation module 520 can generate workout recommendations 560, including up next workout recommendations 562.

In some implementations, recommendation module 520 can include similar workouts recommendation module 540. For example, similar workouts recommendation module 540 can recommend workouts that are similar to workouts that the user has previously performed. Similar workouts recommendation module 540 can recommend workouts from workout catalogue database 506 that are similar to internal workouts provided by workout application 510, previously performed by the user and recorded in user workout database 504. Similar workouts recommendation module 540 can recommend workouts from workout catalogue database 506 that are similar to external workouts previously performed by the user outside of workout application 510 and described in user activity database 502.

In some implementations, similar workouts recommendation module 540 can include internal workout match module 542. For example, internal workout match module 542 can select workouts that are similar to workouts that the user has already performed through workout application 510. Module 542 can select workouts from workout catalogue database 506 that meet certain criteria. For example, module 542 can analyze user workout database 504 to determine the trainer/workout type/duration combinations that appear most frequently among the workouts previously performed by the user in a preceding period of time (e.g., last 60 days, last 45 days, last 30 days, etc.). For a previously performed workout to qualify for this analysis, the user should have completed at least 50% of the previously performed workout. Module 542 can select workouts from workout catalogue database 506 that match a top number (e.g., 1, 2, 3, etc.) of the most frequently occurring trainer/workout type/duration combinations in user workout database 504. For example, module 542 can determine the one most frequently occurring trainer/workout type/duration combinations in user workout database 504 and select a number (e.g., 1, 2, 3, etc.) of workouts from workout catalogue database 506 that match the one most frequently occurring trainer/workout type/duration combination. Module 542 can determine the top three most frequently occurring trainer/workout type/duration combinations in user workout database 504 and select a number (e.g., 1, 2, 3, etc.) of workouts from workout catalogue database 506 that match the top three most frequently occurring trainer/workout type/duration combinations.

In some implementations, similar workouts recommendation module 540 can include external workout match module 544. For example, external workout match module 544 can select workouts that are similar to workouts that the user has already performed external to workout application 510 and described in user activity database 502 (e.g., based on analysis of motion of sensor data, location data, weather data, etc.). Module 544 can analyze qualified workouts in user activity database to determine workout types that the user typically performs external to workout application 510. Module 544 can then select workouts from workout catalogue database 506 that match the determined workout types.

To qualify for analysis by external workout module 544, external workouts should be completed in a preceding period of time (e.g., last 60 days, last 45 days, last 30 days, etc.), be of a duration longer than a threshold period of time (e.g., 5 minutes, 7 minutes, 10 minutes, etc.), and be of a workout type not performed by the user through workout application 510. Additionally, a workout type upon which a workout is selected for recommendation should be performed at least a threshold percentage (e.g., 15%, 20%, 25%, etc.) of the time as compared to other workout types. Thus, if the user performs 100 qualified external workouts and 15% is the threshold, the user should do 15 runs exclusively outside of workout application 510 for the run workout type to be a valid workout type upon which to base a recommended workout selection. Further, the workout type that forms the basis of the recommended workout selection should be a workout type supported by workout application 510. After a workout type has been identified by analyzing the external workout data in user activity database 502 according to the criteria described above, external workout matching module 544 can select a number of workouts from workout catalogue database 506 that match the identified workout type as workout recommendations for the user.

In some implementations, similar workouts recommendation module 540 can receive the workout identifiers and selection reasons for workouts selected by module 542 and/or module 544 and generate similar workout recommendations 564. For example, the selection reasons generated by internal workout matching module 542 can indicate that the selected workout has attributes that match workouts that the user most frequently performs. The selection reasons generated by external workout matching module 534 can indicate that the selected workout has some of the same attributes as workouts that the user performs outside of workout application 510. Similar workouts recommendations 564 can be provided to recommendation module 520 so that recommendation module 520 can generate workout recommendations 560, including similar workout recommendations 564.

In some implementations, recommendation module 520 can include dissimilar workouts recommendation module 550. For example, dissimilar workouts recommendation module 540 can recommend workouts that may have some similar attributes of previous workouts performed by the user but that are different in workout type and/or trainer to workouts that the user has previously performed. For example, dissimilar workouts recommendation module 540 can recommend workouts from workout catalogue database 506 that are similar to internal workouts in workout type and duration performed by the user through workout application 510 but presented by different trainers. Dissimilar workouts recommendation module 540 can recommend workouts from workout catalogue database 506 that have a different training effect or focus area than internal and/or external workouts previously performed by the user.

In some implementations, dissimilar workouts recommendation module 550 can include new trainer module 552. For example, new trainer module 552 can recommend workouts from workout catalogue database 506 that are similar to internal workouts in workout type and duration performed by the user through workout application 510 but presented by different trainers. For example, new trainer module 552 can select workouts for recommendation to the user where the trainers presenting the workout content or leading the workout are different than trainers of previous workouts the user has performed. New trainer module 552 can analyze all workouts in user workout database 504 to determine the trainers associated with each of the workouts that the user has previously performed (e.g., completed, initiated, incomplete, etc.).

New trainer module 552 can analyze qualified workouts previously performed by the user and recorded in user workout database 504 to determine the most frequently performed workout type/duration combinations. For example, the duration can be an average duration of the most frequently performed workouts of the same workout type. A previously performed workout is a qualified workout if the workout was completed within a preceding period of time (e.g., the last 60 days, the last 45 days, the last 30 days, etc.). A previously performed workout is a qualified workout if the user completed more than a threshold amount (e.g., 50%, 60%, 45%, etc.) of the workout.

New trainer module 552 can select a workout from workout catalogue database 506 for recommendation to the user if the workout matches the most frequently performed workout type/duration combinations (e.g., matches the top 1, 2, 3 most frequently performed workout type/duration combinations) and the workout is associated with a trainer is not associated with any previously performed workouts recorded in user workout database 504. As with other workout recommendation modules described herein, the duration of a selected workout does not need to be an exact match. A duration match can be made when the duration of selected workout is within a threshold amount of time of the duration of previously performed workouts. New trainer module 552 can select a number (e.g., 3, 4, 5, etc.) of new trainer workouts to recommend to the user, for example. The selected workouts can be of the same workout type/duration combination. The selected workouts can be associated with different workout type/duration combinations.

In some implementations, dissimilar workouts recommendation module 550 can include complementary workout module 554. For example, complementary workout module 554 can analyze previously performed internal workouts in user workout database 504 and previously performed external workouts in user activity database 502 to determine gaps (e.g., categories of workouts the user is not performing) in the user's workout routine and select recommended workouts to fill those gaps. For example, complementary workout module 554 can be configured with workout focus areas corresponding to a training effect that each type of workout has on the user's body when performed by the user. For example, the workout focus areas (e.g., workout categories, training effect, etc.) can include cardiovascular (cardio) workouts, strength workouts, flexibility workouts, and/or core workouts. Each workout focus area can have associated workout types. For example, running, walking, biking, HIIT (high intensity interval training), etc., can fall under the cardio workout focus area.

Complementary workout module 554 can analyze qualified previously performed internal and/or external workouts to determine which workout focus areas are not represented in the user's previously performed workouts and select workouts for recommendation to the user that are associated with the unrepresented workout focus areas. For example, a previously performed workout (e.g., internal and/or external) is qualified when the workout was completed in a preceding period of time (e.g., the last 60 days, the last 45 days, the last 90 days, etc.) and the user performed the workout for more than a threshold period of time (e.g., 5 minutes, 10 minutes, 12 minutes, etc.). For complementary workout module 554 to select any recommended workouts, at least a threshold percentage (e.g., 75%, 80%, 65%, etc.) of all workouts performed should fall into at least one of the configured (e.g., defined) workout focus areas.

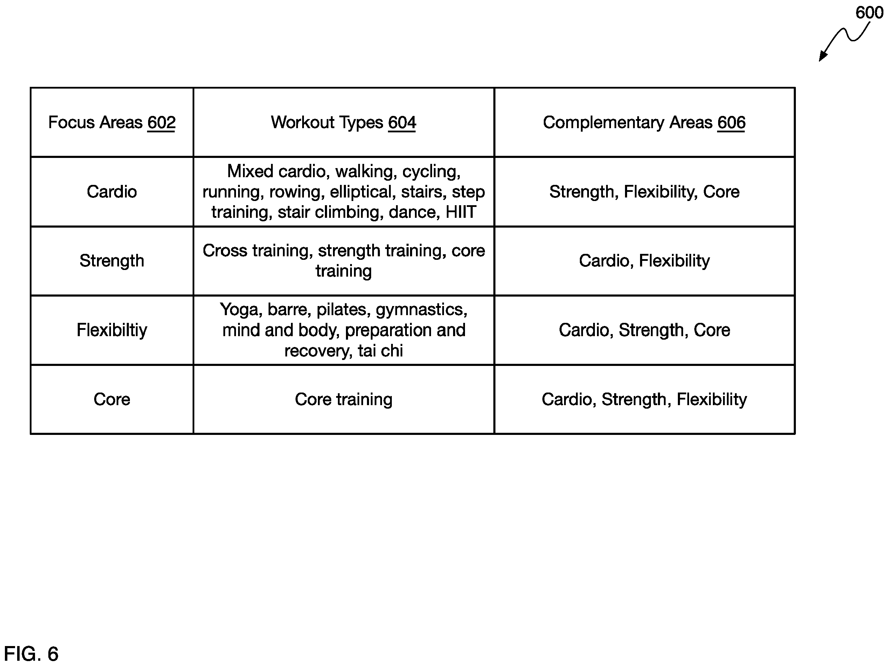

In some implementations, complementary workout module 554 may be configured with a table that defines relationships between workout focus areas, workout types, and complementary workouts. For example, complementary workout module 554 can use the table (e.g., table 600 of FIG. 6) to determine a workout focus area with which a specific workout is associated, and to determine which workout focus area is complementary to previously performed workouts. A workout focus area represented in previously performed workouts may be excluded from selection as a complementary workout focus area. Complementary workout module 554 can select a number (e.g., 3, 4, 5, etc.) of complementary workouts to recommend to the user, for example.

FIG. 6 is an example table 600 associating workout focuses areas with workout types and complementary workout areas. For example, table 600 can be used by complementary workout module 554 to select complementary workouts to recommend to the user.

In some implementations, table 600 can include columns defining workout focus areas 602, workout types 604, and/or complementary workout focus areas 606. Table 600 can include rows that define associations between focus areas 602, workout types 604, and/or complementary areas 606. Thus, according to table 600, the cardio focus area is associated with walking, cycling, elliptical machine, dance, HIIT (high intensity interval training), etc. and the complementary workout focus areas for cardio are strength, flexibility, and core. If the user has previously performed walking and/or running workouts associated with the cardio focus, then complementary workout module 554 can select workouts associated with one of the complementary areas associated with cardio as defined by column 606 (e.g., strength, flexibility, core, etc.).

Similarly, the strength focus area may be associated with cross training, strength training, core training, etc., workouts and may be associated with core and flexibility complementary areas. The flexibility focus area may be associated with yoga, barre, pilates, tai chi, etc., workouts and may be associated with cardio, strength, and core complementary areas. The core focus area may be associated with core training workouts and may be associated with cardio, strength, and flexibility complementary areas.

As described above, complementary workout module 554 may analyzed internal and external workouts to determine the types of workouts that the user has previously performed. Based on the type (e.g., running, cross training, yoga, HIIT, etc.) of workout, complementary workout module 554 can determine the focus area associated with the workout. For example, complementary workout module 554 can determine the focus area (e.g., workout category) based on workout metadata for the workout. Complementary workout module 554 can determine the focus area (e.g., workout category) based table 600 by determining the row in which the workout appears in column 604 and finding the focus area in the same row in column 602. Complementary workout module 554 can determine the complementary area (e.g., workout category) based table 600 by determining the row in which the workout appears in column 604 (or row in which the determined focus area appears in column 602) and finding the complementary focus areas in the same row in column 606.

As illustrated by FIG. 6, the complementary focus areas associated with a performed workout focus area can include several complementary focus areas. For example, the flexibility focus area is associated with complementary focus areas that include cardio, strength, and core focus areas. Complementary workout module 554 can select complementary focus areas from which to recommend workouts by excluding focus areas associated with workouts that the user has previously performed. Thus, complementary workout module 554 is attempting to recommend workouts that are complementary to the flexibility focus area and the user has previously performed strength and cardio workouts as well, then complementary workout module 554 may select workouts from the remaining core workout focus area to recommend to the user.

Complementary workout module 554 can use table 600 to determine specific types of workouts to recommend to the user. For example, if complementary workout module 554 has determined that core is a complementary focus area from which workout recommendations should be selected, complementary workout module 554 can look up the core focus area in column 602 to determine the row in which the core focus area appears. Complementary workout module 554 can then find the workout types corresponding to the core focus area in the same row of column 604. Complementary workout module 554 can then select workouts for recommendation to the user from the workouts associated with the workout types in the determined row of column 604.

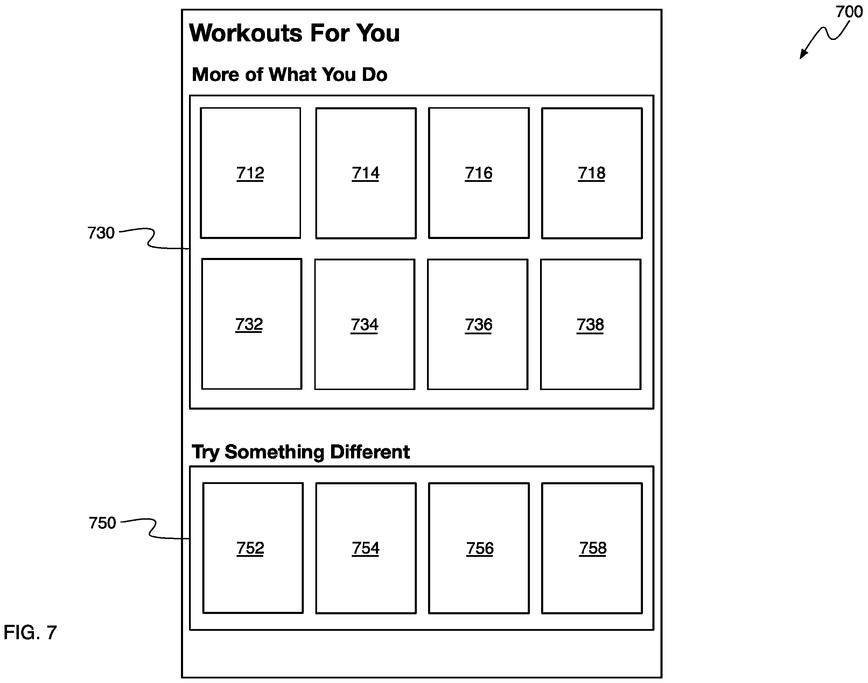

FIG. 7 illustrates an example graphical user interface 700 for presenting recommended workouts. For example, graphical user interface (GUI) 700 can be presented by workout application 112/132/152 of FIG. 1 in response to receiving an invocation of workout application 112/132/152 or in response to workout application 112/132/152 receiving input requesting that workout application 112/132/152 present GUI 700.

In some implementations, GUI 700 can include a "more of what you do" workout recommendation area 730. For example, workout recommendation area 730 can present graphical objects (e.g., graphical objects 712, 714, 716, 718) representing workouts selected for recommendation to the user by up next recommendation module 530 of FIG. 5. Workout recommendation area 730 can include, for example, a number (e.g., 3, 2, 1, etc.) of workouts (e.g., graphical objects 714, 716, 718, etc.) that are next in a workout program, as selected by next in collection module 532. Workout recommendation area 730 can include, for example, a number (e.g., 1, 2, 3, etc.) of workouts (e.g., graphical object 712) that match attributes of the last workout performed by the user, as selected by last workout match module 534.

Each of the graphical objects 712, 714, 716 and/or 718 can present, for the corresponding selected workout, an image representing the workout, an identifier (e.g., title) of the workout, and/or a reason why the workout was selected. For example, the reason can describe that the workout is the next workout in a workout program partially completed by the user. The reason can describe that the workout matches specified attributes of the last workout performed by the user. Thus, the user can view not only recommended workouts personalized to the user, but the user can view a description of why a specific workout is recommended in workout recommendation area 730.

In some implementations, workout recommendation area 730 can present graphical objects (e.g., graphical objects 732, 734, 736, 738) representing workouts selected for recommendation to the user by similar workouts recommendation module 540 of FIG. 5. Workout recommendation area 730 can include, for example, a number (e.g., 3, 2, 1, etc.) of workouts (e.g., graphical objects 736, 738, etc.) that match attributes of previously performed internal workouts, as selected by internal workout matching module 842. Workout recommendation area 730 can include, for example, a number (e.g., 1, 2, 3, etc.) of workouts (e.g., graphical objects 732, 374, etc.) that match attributes of previously performed external workouts, as selected by external workout matching module 534.

Each of the graphical objects 732, 734, 736 and/or 738 can present, for the corresponding selected workout, an image representing the workout, an identifier (e.g., title) of the workout, and/or a reason why the workout was selected. For example, the reason can describe that the workout matches specified attributes of the most frequently performed internal and/or external workouts performed by the user. Thus, the user can view not only recommended workouts personalized to the user, but the user can view a description of why a specific workout is recommended in workout recommendation area 730.

In some implementations, GUI 700 can include an "try something new" workout recommendation area 750. For example, workout recommendation area 750 can present graphical objects (e.g., graphical objects 752, 754, 756, 758) representing workouts selected for recommendation to the user by dissimilar workouts recommendation module 550 of FIG. 5. Workout recommendation area 750 can include, for example, a number (e.g., 3, 2, 1, etc.) of workouts (e.g., graphical objects 756, 758, etc.) similar to workouts previously performed by the user but that are associated with a trainer that the user has never seen before, as selected by new trainer module 552. Workout recommendation area 750 can include, for example, a number (e.g., 1, 2, 3, etc.) of workouts (e.g., graphical objects 752, 754, etc.) that are complementary to internal and/or external workouts that the user has previously performed, as selected by complementary workout module 554.

Each of the graphical objects 752, 754, 756 and/or 758 can present, for the corresponding selected workout, an image representing the workout, an identifier (e.g., title) of the workout, and/or a reason why the workout was selected. For example, the reason can describe that the workout matches specified attributes of the previous workouts performed by the user but introduces the user to a new trainer. The reason can describe that the workout is complementary to the previous internal and/or external workouts performed by the user and introduces the user to a new mode of exercise. Thus, the user can view not only recommended workouts personalized to the user, but the user can view a description of why a specific workout is recommended in workout recommendation area 750.

### Example Processes

To enable the reader to obtain a clear understanding of the technological concepts described herein, the following processes describe specific steps performed in a specific order. However, one or more of the steps of a particular process may be rearranged and/or omitted while remaining within the contemplated scope of the technology disclosed herein. Moreover, different processes, and/or steps thereof, may be combined, recombined, rearranged, omitted, and/or executed in parallel to create different process flows that are also within the contemplated scope of the technology disclosed herein. Additionally, while the processes below may omit or briefly summarize some of the details of the technologies disclosed herein for clarity, the details described in the paragraphs above may be combined with the process steps described below to get a more complete and comprehensive understanding of these processes and the technologies disclosed herein.

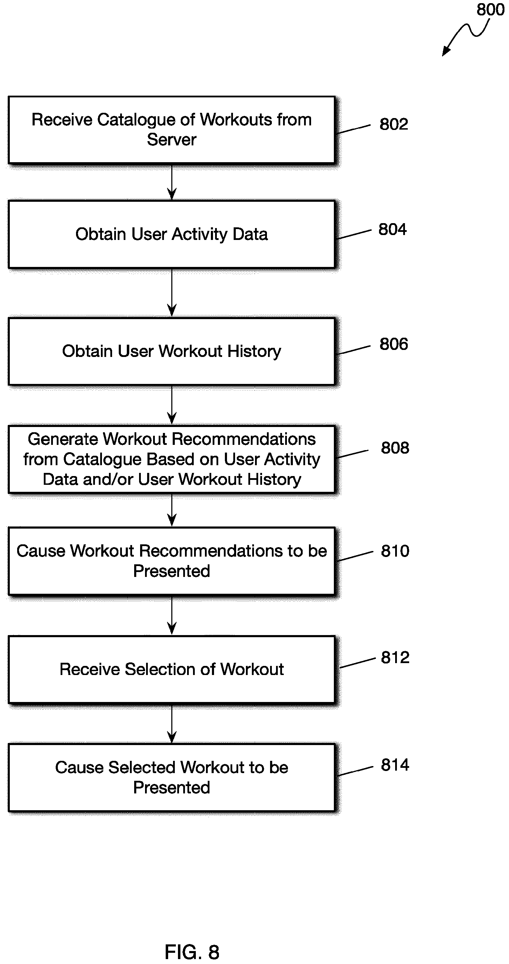

FIG. 8 is flow diagram of an example process 800 for privacy preserving personalized workout recommendations. For example, process 800 can be performed by a personal user device, such as wearable device 110 and/or user playback device 130 of FIG. 1. Process 800 can be performed on the user device (e.g., not a communal device) so that the user's private, personal information can be protected from access from unauthorized users and/or malicious actors who may gain access to the user's personal, private information if the user's information were transmitted to and/or stored on a network server. Process 800 will be described below with reference to wearable device 110 and/or workout application 112/510, however process 800 may be performed by user playback device 130 or some other personal user device (e.g., not a communal device).

At step 802, wearable device 110 can receive a catalogue of workouts from server device 160. For example, the catalogue of workouts can identify and/or describe workouts available through workout service 162 on server device 160. The catalogue of workouts can include metadata that describes various attributes of the workouts provided by workout service 162, as described above. The attributes for each workout can include, among other things, a workout identifier, workout duration, workout type, workout category, trainer identifiers, and other attributes, as may be described herein.

At step 804, wearable device 110 can obtain user activity data associated with the user of wearable device 110. For example, wearable device 110 can obtain user activity data from a local database (e.g., user activity database 116) on wearable device 110 that has been synchronized with user activity databases (e.g., user activity database 136) on other user devices associated with the same user. Thus, the user activity database may include external workout data representing external workouts performed with and/or detected by wearable device 110 and other user devices (e.g., user playback device 130) associate with the same user, as described above.

At step 806, wearable device 110 can obtain user workout history data. For example, the user workout history data can describe workouts presented by workout application 112/154 and provided by workout service 162. Wearable device 110 can obtain user workout history data from a local database (e.g., user workout database 118) on wearable device 110 that has been synchronized with user workout databases (e.g., user workout database 138) on other user devices associated with the same user. Thus, the user workout database may include internal workout data representing internal workouts performed through workout application 112 on wearable device 110 and other workout applications (e.g., workout application 132) on other user devices (e.g., user playback device 130) associate with the same user, as described above.

At step 808, wearable device 110 can generate workout recommendations from the received workout catalogue based on the user activity data and/or the user workout history. For example, recommendation module 520 of workout application 112/132/510 can generate "up next" recommendations, similar workout recommendations, and dissimilar workout recommendations, as described above.

At step 810, wearable device 110 can cause the generated workout recommendations to be presented. For example, wearable device 110 can present the workout recommendations on a display of wearable device and/or through speakers of wearable device 110. Workout recommendations can be presented using audio and video, video-only, or audio only. Wearable device 110 can cause the workout recommendations to be presented by another computing device, such as communal playback device 150, as described above.

At step 812, wearable device 110 can receive a selection of a workout. For example, wearable device 110 can receive user input selecting a recommended workout, or other workout, provided by workout service 162 through workout application 112/132/510.

When wearable device 110 sends workout recommendations to another computing device, such as communal playback device 150, for presentation to the user, the other computing device may receive user input selecting a recommended workout, or other workout, provided by workout service 162 through workout application 112/132/510. Thus, when wearable device 110 sends workout recommendations to communal playback device 150 for presentation to the user, steps 812 and 814 may be performed by communal playback device 150.

At step 814, wearable device 110 can cause the content of the selected workout to be presented. For example, wearable device 110 can cause the content of the selected workout (e.g., recommended workout) to be presented on a display and/or through speakers of wearable device 110. For example, wearable device 110 can obtain the workout identifier for the selected workout, send the workout identifier to workout service 162 in a request for the corresponding workout content, receive the workout content corresponding to the workout identifier, and present the received workout using the display and/or speakers of wearable device 110. When wearable device 110 has a small display, or no display, wearable device 110 may request and/or present an audio-only version of the workout content through the speakers of wearable device 110.

When wearable device 110 sends workout recommendations to another computing device, such as communal playback device 150, for presentation to the user, communal playback device 150 can cause the content of the selected workout (e.g., recommended workout) to be presented on a display and/or through speakers of communal playback device 150. For example, if communal playback device 150 is a set-top-box or other streaming device connected to a television, communal playback device 150 can cause the content of the selected workout (e.g., recommended workout) to be presented on a display and/or through speakers of the connected television. For example, communal playback device 150 can obtain the workout identifier for the selected workout, send the workout identifier to workout service 162 in a request for the corresponding workout content, receive the workout content corresponding to the workout identifier, and present the received workout using the display and/or speakers of communal playback device 150. As the workout presented by communal playback device 150, wearable device 110 can receive workout data describing the user's participation in the workout from communal playback device 150 and store the workout data in user workout database 128 so that the user's workout can be used to generate future workout recommendations.

While the above disclosure describes specific workout attributes based upon which workout recommendations are generated by workout application 112/132/510, other workout attributes may be used to make workout recommendations. For example, system 100 may keep track of statistics (e.g., average, median, range, percentiles, etc.) related to the number of calories burned when performing a particular workout by a community of users who performed the particular workout. Workout application 112/132/510 can recommend the particular workout to the user based on a calorie burn for the user and the calorie burn statistics for the particular workout. For example, if the user's calorie burn goal falls within the range, near the average, etc., of the calorie burn statistics of the particular workout, workout application 112/132/510 can recommend the particular workout to the user.

As another example, system 100 may keep track of statistics (e.g., average, median, range, percentiles, etc.) related to heartrate when performing a particular workout by a community of users who performed the particular workout. Workout application 112/132/510 can recommend the particular workout to the user based on a an average or target heartrate for the user while exercising and the calorie burn statistics for the particular workout. For example, if the user's heartrate target falls within the range, near the average, etc., of the heartrate statistics of the particular workout, workout application 112/132/510 can recommend the particular workout to the user.

Another example workout attribute can include a skill level associated with workouts and/or the user. For example, workout application 112/132/510 may select workouts for recommendation to the user based on a skill level associated with each workout and a skill level determined for the user (e.g., based on explicit user input or derived from the skill levels of previous workouts).

Another example workout attribute can include muscle groups associated with or focused on during workouts. For example, workout application 112/132/510 may select workouts for recommendation to the user based on a muscle groups associated with each workout and muscle groups typically worked by the user in previous workouts. A workout recommendation can be generated by selecting workouts associated with muscle groups that the user has not typically focused on during previous workouts.

### Graphical User Interfaces

This disclosure above describes various Graphical User Interfaces (GUIs) for implementing various features, processes or workflows. These GUIs can be presented on a variety of electronic devices including but not limited to laptop computers, desktop computers, computer terminals, television systems, tablet computers, e-book readers, smart phones, smart watches, wearable devices, and/or other computing devices. One or more of these electronic devices can include a touch-sensitive surface. The touch-sensitive surface can process multiple simultaneous points of input, including processing data related to the pressure, degree or position of each point of input. Such processing can facilitate gestures with multiple fingers, including pinching and swiping.

When the disclosure refers to "select" or "selecting" user interface elements in a GUI, these terms are understood to include clicking or "hovering" with a mouse or other input device over a user interface element, or touching, tapping or gesturing with one or more fingers or stylus on a user interface element. User interface elements can be virtual buttons, menus, selectors, switches, sliders, scrubbers, knobs, thumbnails, links, icons, radio buttons, checkboxes and any other mechanism for receiving input from, or providing feedback to a user.

### Privacy

As described above, one aspect of the present technology is the gathering and use of data available from various sources to improve workout recommendations for a user. The present disclosure contemplates that in some instances, this gathered data may include personal information data that uniquely identifies or can be used to contact or locate a specific person. Such personal information data can include demographic data, location-based data, telephone numbers, email addresses, twitter ID's, home addresses, data or records relating to a user's health or level of fitness (e.g., vital signs measurements, medication information, exercise information), date of birth, or any other identifying or personal information.

The present disclosure recognizes that the use of such personal information data, in the present technology, can be used to the benefit of users. For example, the personal information data can be used to deliver personalized workout content that is of greater interest to the user. Accordingly, use of such personal information data enables users greater control of the delivered workout content. Further, other uses for personal information data that benefit the user are also contemplated by the present disclosure. For instance, health and fitness data may be used to provide insights into a user's general wellness, or may be used as positive feedback to individuals using technology to pursue wellness goals.

The present disclosure contemplates that the entities responsible for the collection, analysis, disclosure, transfer, storage, or other use of such personal information data will comply with well-established privacy policies and/or privacy practices. In particular, such entities should implement and consistently use privacy policies and practices that are generally recognized as meeting or exceeding industry or governmental requirements for maintaining personal information data private and secure. Such policies should be easily accessible by users, and should be updated as the collection and/or use of data changes. Personal information from users should be collected for legitimate and reasonable uses of the entity and not shared or sold outside of those legitimate uses. Further, such collection/sharing should occur after receiving the informed consent of the users. Additionally, such entities should consider taking any needed steps for safeguarding and securing access to such personal information data and ensuring that others with access to the personal information data adhere to their privacy policies and procedures. Further, such entities can subject themselves to evaluation by third parties to certify their adherence to widely accepted privacy policies and practices. In addition, policies and practices should be adapted for the particular types of personal information data being collected and/or accessed and adapted to applicable laws and standards, including jurisdiction-specific considerations. For instance, in the US, collection of or access to certain health data may be governed by federal and/or state laws, such as the Health Insurance Portability and Accountability Act (HIPAA); whereas health data in other countries may be subject to other regulations and policies and should be handled accordingly. Hence different privacy practices should be maintained for different personal data types in each country.

Despite the foregoing, the present disclosure also contemplates embodiments in which users selectively block the use of, or access to, personal information data. That is, the present disclosure contemplates that hardware and/or software elements can be provided to prevent or block access to such personal information data. For example, in the case of workout content delivery services and/or workout recommendations, the present technology can be configured to allow users to select to "opt in" or "opt out" of participation in the collection of personal information data during registration for services or anytime thereafter. In addition to providing "opt in" and "opt out" options, the present disclosure contemplates providing notifications relating to the access or use of personal information. For instance, a user may be notified upon downloading an app that their personal information data will be accessed and then reminded again just before personal information data is accessed by the app.

Moreover, it is the intent of the present disclosure that personal information data should be managed and handled in a way to minimize risks of unintentional or unauthorized access or use. Risk can be minimized by limiting the collection of data and deleting data once it is no longer needed. In addition, and when applicable, including in certain health related applications, data de-identification can be used to protect a user's privacy. De-identification may be facilitated, when appropriate, by removing specific identifiers (e.g., date of birth, etc.), controlling the amount or specificity of data stored (e.g., collecting location data a city level rather than at an address level), controlling how data is stored (e.g., aggregating data across users), and/or other methods.

Therefore, although the present disclosure broadly covers use of personal information data to implement one or more various disclosed embodiments, the present disclosure also contemplates that the various embodiments can also be implemented without the need for accessing such personal information data. That is, the various embodiments of the present technology are not rendered inoperable due to the lack of all or a portion of such personal information data. For example, workout content can be recommended, selected, and delivered to users by inferring preferences based on non-personal information data or a bare minimum amount of personal information, such as the content being requested by the device associated with a user, other non-personal information available to the workout delivery services, or publicly available information.

### Example System Architecture

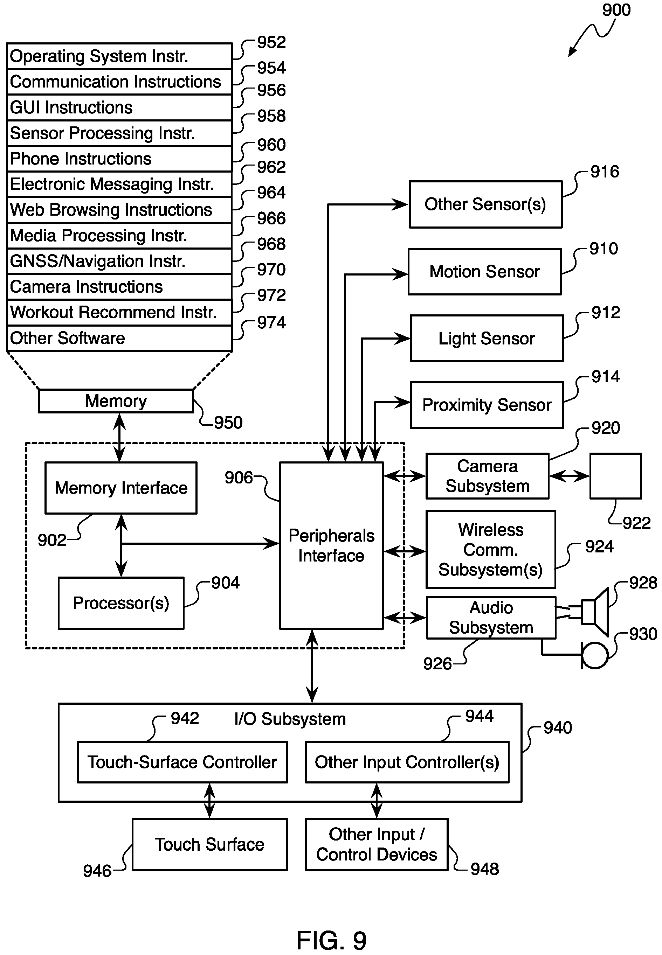

FIG. 9 is a block diagram of an example computing device 900 that can implement the features and processes of FIGS. 1-8. The computing device 900 can include a memory interface 902, one or more data processors, image processors and/or central processing units 904, and a peripherals interface 906. The memory interface 902, the one or more processors 904 and/or the peripherals interface 906 can be separate components or can be integrated in one or more integrated circuits. The various components in the computing device 900 can be coupled by one or more communication buses or signal lines.

Sensors, devices, and subsystems can be coupled to the peripherals interface 906 to facilitate multiple functionalities. For example, a motion sensor 910, a light sensor 912, and a proximity sensor 914 can be coupled to the peripherals interface 906 to facilitate orientation, lighting, and proximity functions. Other sensors 916 can also be connected to the peripherals interface 906, such as a global navigation satellite system (GNSS) (e.g., GPS receiver), a temperature sensor, a biometric sensor, magnetometer or other sensing device, to facilitate related functionalities.

A camera subsystem 920 and an optical sensor 922, e.g., a charged coupled device (CCD) or a complementary metal-oxide semiconductor (CMOS) optical sensor, can be utilized to facilitate camera functions, such as recording photographs and video clips. The camera subsystem 920 and the optical sensor 922 can be used to collect images of a user to be used during authentication of a user, e.g., by performing facial recognition analysis.

Communication functions can be facilitated through one or more wireless communication subsystems 924, which can include radio frequency receivers and transmitters and/or optical (e.g., infrared) receivers and transmitters. The specific design and implementation of the communication subsystem 924 can depend on the communication network(s) over which the computing device 900 is intended to operate. For example, the computing device 900 can include communication subsystems 924 designed to operate over a GSM network, a GPRS network, an EDGE network, a Wi-Fi or WiMax network, and a Bluetooth^{™} network. In particular, the wireless communication subsystems 924 can include hosting protocols such that the device 100 can be configured as a base station for other wireless devices.

An audio subsystem 926 can be coupled to a speaker 928 and a microphone 930 to facilitate voice-enabled functions, such as speaker recognition, voice replication, digital recording, and telephony functions. The audio subsystem 926 can be configured to facilitate processing voice commands, voiceprinting and voice authentication, for example.

The I/O subsystem 940 can include a touch-surface controller 942 and/or other input controller(s) 944. The touch-surface controller 942 can be coupled to a touch surface 946. The touch surface 946 and touch-surface controller 942 can, for example, detect contact and movement or break thereof using any of a plurality of touch sensitivity technologies, including but not limited to capacitive, resistive, infrared, and surface acoustic wave technologies, as well as other proximity sensor arrays or other elements for determining one or more points of contact with the touch surface 946.

The other input controller(s) 944 can be coupled to other input/control devices 948, such as one or more buttons, rocker switches, thumb-wheel, infrared port, USB port, and/or a pointer device such as a stylus. The one or more buttons (not shown) can include an up/down button for volume control of the speaker 928 and/or the microphone 930.

In one implementation, a pressing of the button for a first duration can disengage a lock of the touch surface 946; and a pressing of the button for a second duration that is longer than the first duration can turn power to the computing device 900 on or off. Pressing the button for a third duration can activate a voice control, or voice command, module that enables the user to speak commands into the microphone 930 to cause the device to execute the spoken command. The user can customize a functionality of one or more of the buttons. The touch surface 946 can, for example, also be used to implement virtual or soft buttons and/or a keyboard.

In some implementations, the computing device 900 can present recorded audio and/or video files, such as MP3, AAC, and MPEG files. In some implementations, the computing device 900 can include the functionality of an MP3 player, such as an iPod^{™}.

The memory interface 902 can be coupled to memory 950. The memory 950 can include high-speed random-access memory and/or non-volatile memory, such as one or more magnetic disk storage devices, one or more optical storage devices, and/or flash memory (e.g., NAND, NOR). The memory 950 can store an operating system 952, such as Darwin, RTXC, LINUX, UNIX, OS X, WINDOWS, or an embedded operating system such as VxWorks.

The operating system 952 can include instructions for handling basic system services and for performing hardware dependent tasks. In some implementations, the operating system 952 can be a kernel (e.g., UNIX kernel). In some implementations, the operating system 952 can include instructions for performing privacy preserving personalized workout recommendations. For example, operating system 952 can implement the privacy preserving personalized workout recommendation features as described with reference to FIGS. 1-8.

The memory 950 can also store communication instructions 954 to facilitate communicating with one or more additional devices, one or more computers and/or one or more servers. The memory 950 can include graphical user interface instructions 956 to facilitate graphic user interface processing; sensor processing instructions 958 to facilitate sensor-related processing and functions; phone instructions 960 to facilitate phone-related processes and functions; electronic messaging instructions 962 to facilitate electronic-messaging related processes and functions; web browsing instructions 964 to facilitate web browsing-related processes and functions; media processing instructions 966 to facilitate media processing-related processes and functions; GNSS/Navigation instructions 968 to facilitate GNSS and navigation-related processes and instructions; and/or camera instructions 970 to facilitate camera-related processes and functions.

The memory 950 can store software instructions 972 to facilitate other processes and functions, such as the privacy preserving personalized workout recommendations processes and functions as described with reference to FIGS. 1-8.

The memory 950 can also store other software instructions 974, such as web video instructions to facilitate web video-related processes and functions; and/or web shopping instructions to facilitate web shopping-related processes and functions. In some implementations, the media processing instructions 966 are divided into audio processing instructions and video processing instructions to facilitate audio processing-related processes and functions and video processing-related processes and functions, respectively.

Each of the above identified instructions and applications can correspond to a set of instructions for performing one or more functions described above. These instructions need not be implemented as separate software programs, procedures, or modules. The memory 950 can include additional instructions or fewer instructions. Furthermore, various functions of the computing device 900 can be implemented in hardware and/or in software, including in one or more signal processing and/or application specific integrated circuits.

To aid the Patent Office and any readers of any patent issued on this application in interpreting the claims appended hereto, applicants wish to note that they do not intend any of the appended claims or claim elements to invoke 35 U.S.C. 112(f) unless the words "means for" or "step for" are explicitly used in the particular claim.

## Claims

1. A method comprising:
obtaining, by a workout application on a computing device, user activity data describing external workouts performed by a user of the computing device and distinct from the workout application;
obtaining, by the workout application, a catalogue of workouts provided by a workout service associated with the workout application;
generating, by the workout application, workout recommendations from the catalogue of workouts based on the external workouts performed by the user;
causing, by the workout application, the workout recommendations to be presented on a display.

2. The method of claim 1, further comprising:
determining, by the workout application, a workout type corresponding to the external workouts; and
selecting, by the workout application, a particular workout from the workout catalogue based on the workout type; and
causing, by the workout application, the particular workout to be presented as a recommended workout.

3. The method of claim 1, further comprising:
determining, by the workout application, a first workout category corresponding to the external workouts; and
selecting, by the workout application, a particular workout from the workout catalogue based on the first workout category, where a second workout category associated with the particular workout corresponds to the first workout category; and
causing, by the workout application, the particular workout to be presented as a recommended workout.

4. The method of claim 1, further comprising:
determining, by the workout application, a plurality of workout categories associated with the external workouts; and
selecting, by the workout application, a particular workout from the workout catalogue based on the plurality of workout categories, where a second workout category associated with the particular workout is distinct from the plurality of workout categories; and
causing, by the workout application, the particular workout to be presented as a recommended workout.

5. The method of claim 1, further comprising:
obtaining, by the workout application, user workout data describing internal workouts previously performed by the user through the workout application;
determining, by the workout application, a plurality of workout categories associated with the external workouts and the internal workouts; and
selecting, by the workout application, a particular workout from the workout catalogue based on the plurality of workout categories, where a second workout category associated with the particular workout is distinct from the plurality of workout categories; and
causing, by the workout application, the particular workout to be presented as a recommended workout.

6. The method of claim 1, further comprising:
determining, by the workout application, a first workout category corresponding to the external workouts;
obtaining, by the workout application, user workout data describing internal workouts previously performed by the user through the workout application;
determining, by the workout application, that the first workout category is not associated with an internal workout described in the user workout data;
in response to determining that the first workout category is not associated with an internal workout described in the user workout data, selecting, by the workout application, a particular workout from the workout catalogue based on the first workout category, where a second workout category associated with the particular workout corresponds to the first workout category; and
causing, by the workout application, the particular workout to be presented as a recommended workout.

7. The method of any preceding claim, further comprising:
causing, by the workout application, the particular workout to be presented as a recommended workout by a playback device that is distinct from the computing device.

8. A computer readable medium including one or more sequences of instructions that, when executed by one or more processors, cause the processors to perform operations comprising:
obtaining, by a workout application on a computing device, user activity data describing external workouts performed by a user of the computing device and distinct from the workout application;
obtaining, by the workout application, a catalogue of workouts provided by a workout service associated with the workout application;
generating, by the workout application, workout recommendations from the catalogue of workouts based on the external workouts performed by the user;
causing, by the workout application, the workout recommendations to be presented on a display.

9. The computer readable medium of claim 8, wherein the instructions cause the processors to perform operations comprising:
determining, by the workout application, a workout type corresponding to the external workouts; and
selecting, by the workout application, a particular workout from the workout catalogue based on the workout type; and
causing, by the workout application, the particular workout to be presented as a recommended workout.

10. The computer readable medium of claim 8, wherein the instructions cause the processors to perform operations comprising:
determining, by the workout application, a first workout category corresponding to the external workouts; and
selecting, by the workout application, a particular workout from the workout catalogue based on the first workout category, where a second workout category associated with the particular workout corresponds to the first workout category; and
causing, by the workout application, the particular workout to be presented as a recommended workout.

11. The computer readable medium of claim 8, wherein the instructions cause the processors to perform operations comprising:
determining, by the workout application, a plurality of workout categories associated with the external workouts; and
selecting, by the workout application, a particular workout from the workout catalogue based on the plurality of workout categories, where a second workout category associated with the particular workout is distinct from the plurality of workout categories; and
causing, by the workout application, the particular workout to be presented as a recommended workout.

12. The computer readable medium of claim 8, wherein the instructions cause the processors to perform operations comprising:
obtaining, by the workout application, user workout data describing internal workouts previously performed by the user through the workout application;
determining, by the workout application, a plurality of workout categories associated with the external workouts and the internal workouts; and
selecting, by the workout application, a particular workout from the workout catalogue based on the plurality of workout categories, where a second workout category associated with the particular workout is distinct from the plurality of workout categories; and
causing, by the workout application, the particular workout to be presented as a recommended workout.

13. The computer readable medium of claim 8, wherein the instructions cause the processors to perform operations comprising:
determining, by the workout application, a first workout category corresponding to the external workouts;
obtaining, by the workout application, user workout data describing internal workouts previously performed by the user through the workout application;
determining, by the workout application, that the first workout category is not associated with an internal workout described in the user workout data;
in response to determining that the first workout category is not associated with an internal workout described in the user workout data, selecting, by the workout application, a particular workout from the workout catalogue based on the first workout category, where a second workout category associated with the particular workout corresponds to the first workout category; and
causing, by the workout application, the particular workout to be presented as a recommended workout.

14. The computer readable medium of claim 8, wherein the instructions cause the processors to perform operations comprising:
causing, by the workout application, the particular workout to be presented as a recommended workout by a playback device that is distinct from the computing device.

15. A system comprising:
one or more processors; and
a computer readable medium including one or more sequences of instructions that, when executed by the one or more processors, cause the processors to perform operations comprising a method recited in claims 1-7.
